# EUROPEAN PATENT SPECIFICATION

(11) **EP 2 468 710 B1**
(45) Date of publication and mention of the grant of the patent: **01.03.2017**
(21) Application number: 10809836.9
(22) Date of filing: 30.07.2010
(51) Int. Cl.: C07C 29/82, C07C 31/42

(54) **PROCESS FOR PREPARATION OF HEXAFLUOROACETONE MONOHYDRATE**
VERFAHREN ZUR HERSTELLUNG VON HEXAFLUORACETONMONOHYDRAT
PROCÉDÉ DE PRÉPARATION DU MONOHYDRATE D'HEXAFLUOROACÉTONE

(30) Priority: 18.08.2009 JP 2009189347
(43) Date of publication of application: 27.06.2012
(73) Proprietor: Central Glass Company, Limited, Yamaguchi 755-0001 (JP)
(72) Inventor: KATSUHARA, Yutaka, Kawagoe-shi Saitama 350-1151 (JP); HAYASAKA, Tatsuya, Kawagoe-shi Saitama 350-1151 (JP); WATANABE, Mineo, Kawagoe-shi Saitama 350-1151 (JP); KUME, Takashi, Kawagoe-shi Saitama 350-1151 (JP)
(74) Representative: Manitz, Finsterwald & Partner GbR
(86) International application number: PCT/JP2010/062878
(87) International publication number: WO 2011/021491

(56) References cited:
- WO-A1-2009/028584
- JP-A- 1 203 339
- JP-A- 59 181 235
- JP-A- 2000 063 316
- JP-B- 46 006 761
- US-A- 3 234 172
- US-A- 3 433 838
- US-A- 3 520 929
- GRZEGORZ MLOSTON ET AL.: 'Reactions of 2- Unsubstituted 1H-Imidazole 3-Oxides with 2,2- Bis(trifluoromethyl)ethene-1,1-dicarbonitri le: A Stepwisel,3-Dipolar Cycloaddition' HELVETICA CHIMICA ACTA vol. 89, 2006, pages 1304 - 1316, XP008154202
- DIWAKAR M. PAWAR ET AL.: 'Conformational Equilibria in Formic Acid and the Adduct of Formic Acid and Hexafluoroacetone, HC02C(CF3) 20H' JOURNAL OF ORGANIC CHEMISTRY vol. 72, 2007, pages 2003 - 2007, XP008154203
- W. J. MIDDLETON ET AL.: 'Hydrogen Bonding in Fluoro Alcohols' JOURNAL OF THE AMERICAN CHEMICAL SOCIETY vol. 86, no. 22, 1964, pages 4948 - 4952, XP008154205

## Description

### TECHNICAL FIELD

The present invention relates to a process for producing hexafluoroacetone monohydrate (1,1,1,3,3,3-hexafluoropropane-2-diol) and in more detail to a process of dehydrating a hexafluoroacetone hydrate in the presence of an organic solvent.

### BACKGROUND OF THE INVENTION

Hexafluoroacetone is an important compound as a pharmaceutical intermediate or reaction raw material. Hexafluoroacetone is industrially produced by an epoxidation of hexafluoropropene and a subsequent isomerization, a process by subjecting hexachloroacetone, which is obtained by chlorinating acetone, to a substitution fluorination with hydrogen fluoride by a chromium activated carbon supported catalyst or the like, etc. Hexafluoroacetone is a gas having a boiling point of -28°C under atmospheric pressure. Therefore, for the sake of handling convenience, hexafluoroacetone trihydrate, which can be handled as a composition having a constant boiling point of 106°C, is used as a raw material in many reactions or serves for storage. However, depending on the reaction conditions, the target and other demands, hexafluoroacetone monohydrate is requested in some cases. Hexafluoroacetone monohydrate is known to be capable of introducing a hexafluoroisopropanol group to 5-position of uracil (Non-patent publication 1).

Hexafluoroacetone monohydrate is a crystal having a structure of gem-diol and a melting point of 46°C. Concurrently with melting, it decomposes to make a disproportionation into hexafluoroacetone and hexafluoroacetone trihydrate (Patent Publication 1), and it deliquesces at once by moisture in the air even at low temperatures. Therefore, it is a compound that is very difficult in handling, hardly available, and unstable.

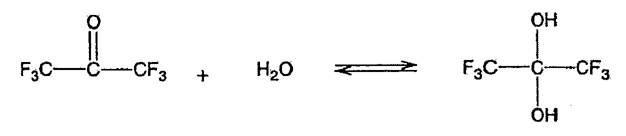

Regarding hexafluoroacetone monohydrate, it is known that hexafluoroacetone monohydrate is obtained as some of hexafluoroacetone and somewhat moist, needle-like white-color crystals that contain water, by allowing hexafluoroacetone to be absorbed into water in two steps (Patent Publication 1).

Furthermore, a description that hexafluoroacetone dissolves in organic solvent (e.g., Patent Publication 1) and a description that an aqueous solution of hexafluoroacetone is extracted with ether (Non-patent Publication 2) are found in publications.

US 3,433,838 discloses a method for producing hexafluoroacetone monohydrate by adding hexafluoroacetone into an aqueous solution of hexafluoroacetone trihydrate.

### PRIOR ART PUBLICATIONS

### PATENT PUBLICATIONS

Patent Publication 1: Specification of US Patent No. 3374273

### NON-PATENT PUBLICATIONS

Non-patent Publication 1: Chemical Abstracts 95: 151089 (All-Union Cancer Res. Cent., Moscow, USSR. Bioorganicheskaya Khimiya (1981), 7(7), 1047-53.)
Non-patent Publication 2: Canadian Journal of Chemistry (1955), 33 453-7.

### SUMMARY OF THE INVENTION AND THE FURTHER DISCLOSURE

(1) As inferred also from Patent Publication 1, a solid hexafluoroacetone monohydrate is formed when equimolar water is added to hexafluoroacetone. Therefore, it is difficult to produce hexafluoroacetone monohydrate in the form of not containing water or hexafluoroacetone trihydrate. (2) In contrast with this, it is general to use a dehydrating agent, such as concentrated sulfuric acid and phosphorus pentoxide, in order to remove excessive water from a hexafluoroacetone hydrate, such as hexafluoroacetone trihydrate, or its aqueous solution. In this case, however, hexafluoroacetone monohydrate is not obtained, but anhydrous hexafluoroacetone is obtained.

Furthermore, as mentioned above, hexafluoroacetone monohydrate is a deliquescent, unstable compound. Therefore, even if hexafluoroacetone monohydrate is dissolved in solvent, there is obtained a solution containing water or hexafluoroacetone trihydrate, resulting in no obtainment of a composition that is made up of hexafluoroacetone monohydrate and solvent and is substantially free from water.

Then, the present disclosure provides a process for preparing hexafluoroacetone monohydrate that is substantially free from water and provides a composition of hexafluoroacetone monohydrate in the form of easy handling.

The present inventors have made a study on the process for producing hexafluoroacetone monohydrate (1,1,1,3,3,3-hexafluoropropane-2-diol) that is substantially free from water, from an aqueous solution containing hexafluoroacetone or a hydrate such as hexafluoroacetone trihydrate. With this, we have found that, upon making hexafluoroacetone monohydrate by adjusting the molar ratio of hexafluoroacetone to water, it exists stably even at melting point of hexafluoroacetone monohydrate or higher by conducting that in the presence of organic solvent, thereby reaching the present invention. Furthermore, it was also found that a composition made up of hexafluoroacetone monohydrate and organic solvent, which was obtained in this production process, was useful for various uses, storage, etc.

The present invention is as follows.
[Invention 1] A process for producing hexafluoroacetone monohydrate, comprising introducing a hexafluoroacetone hydrate and an organic solvent into a distillation column after a previous mixing of them or separately, obtaining by azeotropic distillation (a) a composition containing the organic solvent and water as a low-boiling-point component from a column top, and (b) a composition containing hexafluoroacetone monohydrate and the organic solvent as a high-boiling-point component from a column bottom.
[Invention 2] A production process of Invention 1, wherein at least one of the hexafluoroacetone hydrate and the organic solvent is continuously introduced into the distillation column.
[Invention 3] A production process of Invention 1 or 2, wherein at least one of the compositions obtained from the column bottom or the column top is continuously taken out of the distillation column.
[Invention 4] A production process of Inventions 1-3, wherein the hexafluoroacetone hydrate is hexafluoroacetone trihydrate (HFA·3W).
[Invention 5] A production process of Invention 1, wherein an azeotrope of the hexafluoroacetone monohydrate and the organic solvent is obtained from the composition containing the hexafluoroacetone monohydrate and the organic solvent, which is obtained from the column bottom.
[Invention 6] A production process of any of Inventions 1 to 5, wherein the organic solvent is an aromatic compound or ether compound.

### BRIEF DESCRIPTION OF THE DRAWINGS

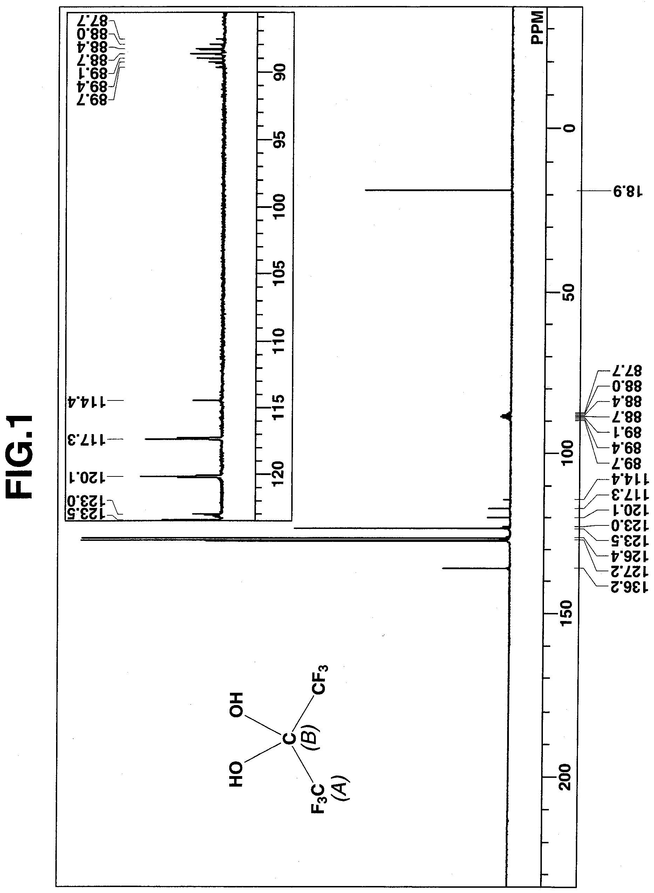

Fig. 1 is a ¹³C-NMR chart of a composition obtained by Example 1.

### DETAILED DESCRIPTION

A production process of the present invention brings an advantageous effect that hexafluoroacetone monohydrate substantially free from water can easily be produced from a hydrate, such as hexafluoroacetone trihydrate, without using a special dehydrating agent or the like.

A composition of the present disclosure comprising hexafluoroacetone monohydrate and an organic solvent brings an advantageous effect that it can be used as a reagent in which hexafluoroacetone monohydrate can be handled as a stable reaction substrate.

In the following, the present invention is explained in detail.

In the claims and the specification, hexafluoroacetone may be represented by "HFA".

In the claims and the specification, hexafluoroacetone monohydrate may be represented by "HFA·W".

In the claims and the specification, hexafluoroacetone trihydrate may be represented by "HFA·3W".

In the claims and the specification, a hexafluoroacetone hydrate refers to a hydrate, in which the number of hydration is not limited, or its aqueous solution, and refers to a concept containing "HFA·3W".

An organic solvent solution of hexafluoroacetone monohydrate of the present disclosure is a composition made up of hexafluoroacetone monohydrate and an organic solvent. As the organic solvent, it is possible to mention aromatic compounds, ether compounds, halogenated solvents, etc., which are liquids at ordinary temperature (about 25°C). The aromatic compounds are not particularly limited and may be either monocyclic, ring-assembled or condensed polycyclic. Among them, monocyclic, that is, benzene or a compound prepared by replacing a hydrogen atom of benzene with a halogen atom, alkyl group, fluoroalkyl group or the like is preferable. As such compound, it is possible to mention benzene, toluene, xylene, ethylbenzene, chlorobenzene, benzotrifluoride, 2,4-dichlorobenzotrifluoride, o-, m- or p-bistrifluoromethylbenzene, etc. As the ether compounds, it is possible to mention chain ethers such as dimethyl ether, diethyl ether, isopropyl ether, ethyl isopropyl ether, butyl methyl ether and ethyl butyl ether, and cyclic ethers such as tetrahydrofuran, pyran and dioxane. As the halogenated solvents, it is possible to mention carbon tetrachloride, chloroform, dichloromethane, 1,2-dichloroethane, cis-1,2-dichloroethylene, trans-1,2-dichloroethylene, and 1,1,2-trichloroethane. The organic solvent can suitably be selected, depending on the purpose of its use, particularly the purpose of the reaction. Furthermore, it is also possible to use at least two of these together. As the organic solvent, aromatic compounds or ether compounds are preferable, and aromatic compounds are more preferable.

In the water addition method, the above-mentioned organic solvents, which form azeotropes with water, are preferable. Hexafluoroacetone monohydrate concentration may be determined such that it becomes a desired concentration. Normally, it is 1-100 parts by mass relative to 1 part by mass of hexafluoroacetone. It can be determined depending on use.

Hexafluoroacetone monohydrate according to the present disclosure can be produced by the after-mentioned water addition method and dehydration method.

Hexafluoroacetone trihydrate is a dihydrate of hexafluoroacetone monohydrate, which is a gem-diol formed by a reaction between hexafluoroacetone and water (In the present specification, two molecules of water of this dihydrate refer to "added water".), and is a chemical substance represented by

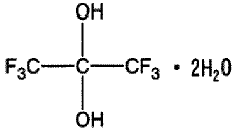

It is a stable liquid having the maximum boiling mixture (boiling point 106°C).

In contrast with this, hexafluoroacetone monohydrate is a solid having a melting point of 46°C. At higher than the melting point, it decomposes to make a disproportionation into anhydrous hexafluoroacetone and hexafluoroacetone trihydrate (Patent Publication 1).

### [Water addition method]

To a mixture of a hexafluoroacetone hydrate, for example, hexafluoroacetone trihydrate, or an aqueous solution containing hexafluoroacetone, and an organic solvent, hexafluoroacetone that is equimolar with water (the added water and free water) in the system is added. With this, it is possible to obtain hexafluoroacetone monohydrate according to the present disclosure under a condition, which is dissolved in the organic solvent.

As a hexafluoroacetone hydrate, it is preferable to use hexafluoroacetone trihydrate, which is easily available. It may be one having a hydration number less than the trihydrate or an aqueous solution of hexafluoroacetone trihydrate. Hexafluoroacetone may be used in an amount that becomes equimolar with the amount of water contained in these hexafluoroacetone-related substances. It is preferable to set hexafluoroacetone/water (the total of the added water and free water) at substantially 1, and it may be set at normally 1-1.2. In view of the operational loss and the like, some of the excessive amount is admissible. In case that hexafluoroacetone is excessive, it is not preferable due to washout. In case that it is insufficient, it is not preferable since free water or hexafluoroacetone trihydrate remains in the organic solvent. In case that water remains, however, it is also possible to make the target hexafluoroacetone monohydrate solution by removing water by the after-mentioned dehydration method.

When introducing hexafluoroacetone into the system, it is preferable to warm up a liquid fraction in the system at a temperature from 40°C to boiling point of the liquid. That is because it is not possible to obtain a sufficient reaction rate at a low temperature. As the reaction proceeds, temperature of the reaction system increases and lowers upon completion of the reaction. Therefore, temperature in the system is set normally to be lower than boiling point (106°C) of hexafluoroacetone trihydrate, although it varies depending on the type of the organic solvent.

The quantitative ratio of the organic solvent to hexafluoroacetone may be determined such that the hexafluoroacetone monohydrate solution has a desired concentration. Normally, it is 1-1000 parts by mass, preferably 1-100 parts by mass, relative to 1 part by mass of hexafluoroacetone. If the organic solvent is insufficient, there occur, depending on the reaction apparatus shape and method, inconveniences such as that stirring becomes difficult by the precipitation of the formed hexafluoroacetone monohydrate during the production and that the removal of generated heat is not smooth. If it is excessive, there is no problem in stirring, but there occur inconveniences such as that the apparatus gets larger and that concentration of the hexafluoroacetone monohydrate solution formed is small. Therefore, either is not preferable.

An example of the procedure of the water addition method is explained. A predetermined amount of hexafluoroacetone trihydrate and an organic solvent appropriate for the purpose are introduced into a sealed reaction vessel equipped with a stirring device and a gas inlet.

As the reaction proceeds rapidly, temperature rises. Therefore, while cooling from outside, a mixture in the reaction vessel is stirred, and hexafluoroacetone in an amount such that the molar ratio of hexafluoroacetone to water (the added water and free water) becomes 1:1 is gradually introduced from the gas inlet of the reaction vessel. Upon this, attention is paid not to clog the gas inlet due to precipitation of hexafluoroacetone monohydrate even if the opening portion is positioned at the gas phase portion or the organic solvent liquid phase portion. Even after the termination of introducing a predetermined amount of hexafluoroacetone, stirring is continued, and the point when the reaction liquid temperature has lowered and the pressure has lowered is judged as the termination of the reaction, thereby obtaining an organic solvent solution of hexafluoroacetone monohydrate with a high yield.

Although the reaction apparatus is not particularly limited, a pressure-proof vessel or a vessel equipped with a condenser is preferable, and one equipped with a stirrer is preferable. Regarding material of the apparatus, it is possible to use stainless steel, nickel alloy steel, glass, fluororesin, carbon, polyethylene, or materials lined or cladded with these materials.

### [Dehydration method]

By removing an excessive amount of water from a mixture of a hexafluoroacetone hydrate, for example, hexafluoroacetone trihydrate, or an aqueous solution containing hexafluoroacetone, and an organic solvent, it is possible to obtain hexafluoroacetone monohydrate according to the present disclosure under a condition that it is dissolved in the organic solvent.

The dehydration method is a method in which the hexafluoroacetone hydrate and the organic solvent, which have been introduced into the vessel, are heated, thereby removing the excessive or free water as a gas phase component and thereby obtaining a composition made up of hexafluoroacetone monohydrate and the organic solvent from the liquid phase fraction. The water to be removed can be removed by forming an azeotrope with the organic solvent.

Normally, this dehydration method is conducted by using an ordinary distillation apparatus equipped with a distillation still, a distillation column, a condenser and other devices. The distillation apparatus may be of any type. As the distillation column, it is possible to use any of simple distillation, packed column, bubble cap column, plate column, etc. The packing material is also not particularly limited. It is possible to use any of Raschig ring, Heli-Pack, Pall Ring, etc. Regarding the distillation mode, it is possible to use any of batch type, half batch type or half continuous type for continuously supplying either the organic solvent or the hexafluoroacetone hydrate, and continuous type. Regarding the material of the apparatus, it is possible to use stainless steel, nickel alloy steel, glass, fluororesin, carbon, polyethylene, or materials lined or cladded with these materials.

The hexafluoroacetone hydrate and the organic solvent appropriate for the purpose are introduced into a distillation apparatus after a previous mixing of them or separately. Herein, the hexafluoroacetone hydrate may previously be subjected to the removal of excessive water by a pretreatment by a chemical substance that is used generally as a dehydrating agent, such as concentrated sulfuric acid, sulfuric anhydride and phosphorus pentoxide, or by an adsorbing agent such as molecular sieve.

In the following, the distillation is explained with respect to the condition in the case of conducting it by batch type. Based on this explanation, a person skilled in the art can easily select the condition in the distillation of batch type or continuous type.

In the distillation, the dehydration is conducted by an azeotropic distillation. Therefore, it is an organic solvent that forms an azeotrope with water. The column top temperature is determined by an azeotropic temperature of water and the organic solvent, and it varies depending on the type of the organic solvent. Furthermore, in order to use a distillation column that is low in distillation efficiency or to increase the operation efficiency, an azeotrope is distilled out in a certain temperature range. Therefore, this temperature range is also referred to as azeotropic temperature.

For example, as an azeotropic temperature of water and the organic solvent, it is possible to mention 69.25°C of water/benzene, 85.0°C of water/toluene, 94.5°C of water/m-xylene, 92°C of water/ethylbenzene, etc. For any of these, it is possible to conduct the distillation operation with a range of about 5°C above and below. By condensing water and the organic solvent, which are recovered from the column top, it is separated into a layer containing the organic solvent as a major component and a layer containing water. The organic solvent can be used again in the method of the present invention. Furthermore, the water-containing layer may contain a hexafluoroacetone hydrate. From this, it is also possible to recover hexafluoroacetone trihydrate and use it again in the method of the present invention.

On the other hand, it suffices that the column bottom temperature is a temperature at which distillation can be maintained. Therefore, it is set at 50°C to 120°C. It is known that hexafluoroacetone monohydrate alone decomposes at 48°C. Even if the column bottom temperature becomes 50°C or higher, the decomposition of hexafluoroacetone monohydrate does not occur, and hexafluoroacetone monohydrate exists stably as an organic solvent solution at the column bottom.

In case that hexafluoroacetone monohydrate and the organic solvent form an azeotrope, it is possible from this hexafluoroacetone monohydrate solution to obtain a composition made up of hexafluoroacetone monohydrate and the organic solvent, which is an azeotrope.

In the dehydration method, the amount of the organic solvent necessary to hexafluoroacetone trihydrate varies depending on its type. It is necessary to use an amount more than that for forming an azeotrope with water to remove it. Furthermore, it is determined such that the hexafluoroacetone monohydrate solution has a desired concentration. Normally, it is 1-1000 parts by mass, preferably 1-100 parts by mass, relative to 1 part by mass of hexafluoroacetone. If the organic solvent is insufficient, the dehydration effect and the distillation operation do not become stable. If it is excessive, there is no problem in the dehydration effect, but it is accompanied by that the utility consumption becomes large, that concentration of hexafluoroacetone monohydrate formed at the column bottom becomes low, and that the apparatus becomes large. Therefore, either is not preferable.

The dehydration can be conducted under reduced pressure or even under a pressurized condition. It is preferable to conduct that at ordinary pressure. In the following, it is explained with respect to the case of conducting under ordinary pressure (0MPa-G (gauge pressure)). To conduct it under other pressure conditions is also included in the scope of the present invention.

If the column bottom temperature is increased to exceed the azeotropic point of water and the organic solvent, vapor is liquefied by the condenser at the column top, and reflux starts. As the reflux liquid is gradually taken out, azeotropy of water and the organic solvent terminates. If distillation is terminated at this point, hexafluoroacetone monohydrate is recovered as an organic solvent solution at the column bottom.

If distillation is further continued, it may lead to observation of a new reflux at a temperature that is determined by the azeotrope of hexafluoroacetone monohydrate and the organic solvent. With this, a mixture of hexafluoroacetone monohydrate and the organic solvent may be distilled out of the column top. Thus, it may be obtained as an azeotrope. In this manner, a component made up of a composition of hexafluoroacetone monohydrate and the organic solvent and a component made up of water and the organic solvent are obtained separately. It is possible to use again in the present process the organic solvent obtained by separating the composition of water and the organic solvent, which has been recovered from the column top, into two layers to separate and remove water.

The dehydration method of the present invention can be applied to a method for producing hexafluoroacetone derivatives. It is possible to use a hexafluoroacetone monohydrate containing no water as a reaction reagent by adding, prior to the reaction, an organic solvent to hexafluoroacetone trihydrate, which is generally easily available, or its aqueous solution in a reaction vessel and then removing the organic solvent and water by an azeotropic distillation by applying the above-mentioned dehydration method. Furthermore, it is possible to conduct a reaction related to a hexafluoroacetone monohydrate containing no water by adding, prior to the start of the reaction, hexafluoroacetone trihydrate, which is generally easily available, or its aqueous solution and an organic solvent into a reaction vessel and then removing the organic solvent and water by an azeotropic distillation by applying the above-mentioned dehydration method during the reaction.

### EXAMPLES

In the following, the present invention is explained in detail by examples, but the present invention is not limited to these examples.

### [Example 1] Water addition method

### (Reference Example)

86g (100mL) of toluene and 22g (0.1 mol) of HFA·3W were put into a 200mL, four-necked, glass, reaction apparatus, which was equipped with a stirring device, a dry ice/acetone reflux condenser, a thermometer and a gas inlet, and in which an opening part of the reflux device was made to be a closed system by a balloon. While cooling the outer side, 32g (0.2 mol) of HFA was gradually introduced from the gas inlet. After introducing a predetermined amount, temperature of the reaction liquid was confirmed to be lower than room temperature. Then, the reaction was terminated, thereby obtaining 139g of a colorless, transparent solution having a specific gravity of 1.05 (yield 98%).

By NMR (¹³C, H, and F) and FT-IR measurements, it was confirmed that only hexafluoroacetone monohydrate
(1,1,1,3,3,3-hexafluoropropane-2-diol) existed in toluene. By measuring the moisture with a Karl Fischer moisture measurement apparatus, it was confirmed that the molar ratio of hexafluoroacetone to water was 1:1. In the Karl Fischer moisture measurement condition, water quantitatively generated from hexafluoroacetone monohydrate
(1,1,1,3,3,3-hexafluoropropane-2-diol) is measured.

A chart of ¹³C-NMR spectrum (standard: d-chloroform, external standard method) is shown in Fig. 1. The peaks at 18.9 δ and 123.5-136.2 ppm are assigned to toluene, and all of the other peaks are assigned to hexafluoroacetone monohydrate.

### [Example 2] Water addition method

### (Reference Example)

Hexafluoroacetone monohydrate was produced by the same method as that of Example 1, except in that 72.5g of isopropyl ether was used in place of toluene. After terminating the reaction, there was obtained 127g of a colorless, transparent solution having a specific gravity of 1.00 (yield 100%).

By NMR (¹³C, H, and F) and FT-IR measurements, it was confirmed that only hexafluoroacetone monohydrate
(1,1,1,3,3,3-hexafluoropropane-2-diol) existed in isopropyl ether. By measuring the moisture with a Karl Fischer moisture measurement apparatus, it was confirmed that the molar ratio of hexafluoroacetone to water was 1:1.

### [Example 3] Dehydration method

A 100mL round-bottom flask equipped with a glass, reflux/distillation column and a 20cm distillation column (void column) was charged with 22g (0.1 mol) of HFA·3W and 38g (0.49 mol) of benzene, followed by heating in an oil bath under stirring with a stirrer. At 69°C-71°C, an azeotrope of water/benzene (it became cloudy upon condensation) was distilled out. At a point when the distillation temperature of the column top increased to 73°C, the distillation was stopped once. The distillate separated into two layers to form a benzene layer as the upper layer and an aqueous layer as the lower layer (benzene layer: 6.8g, specific gravity 0.87, aqueous layer: 3.6g, specific gravity 1.15). Due to the aqueous layer having a specific gravity greater than 1, it was suggested that some of HFA component was mixed therein. As the distillation was further continued, a new azeotrope was distilled out at 73.8°C-74.0°C. A homogeneous, colorless, transparent liquid of 40g and of a specific gravity of 1.04, which had been distilled out, was found, as follows. By NMR (¹³C, H, and F) and FT-IR measurements, it was found that only hexafluoroacetone monohydrate (1,1,1,3,3,3-hexafluoropropane-2-diol) existed. By measuring the moisture with a Karl Fischer moisture measurement apparatus, it was confirmed that the molar ratio of HFA to water was 1:1. An azeotrope was found to be a benzene solution of 39 mass % HFA·W. The residual, homogeneous, colorless, transparent liquid of 4.6g and of a specific gravity of 1.37 was confirmed by NMR (¹³C, H and F) and FT-IR and Karl Fischer moisture measurement to be a benzene solution of 44 mass % HFA·W. Yield of hexafluoroacetone monohydrate by the present dehydration method was 96%.

### [Example 4] Dehydration method.

Hexafluoroacetone monohydrate was produced by the same dehydration method as that of Example 3, except in that 54g of meta-xylene was used in place of benzene. Distilling-out of an azeotrope started at 94°C, the minimum azeotropic temperature of meta-xylene and water. Distilling-out of a liquid becoming cloudy by condensation continued until a distilling-out temperature of 97°C. The distillate separated into two layers to form a meta-xylene layer as the upper layer and an aqueous layer as the lower layer (meta-xylene layer: 1.2g, specific gravity 0.86, aqueous layer: 7.4g, specific gravity 1.37). Due to the aqueous layer having a specific gravity considerably greater than 1, it was suggested that HFA component was mixed therein. The residual, homogeneous, colorless, transparent liquid of 67.4g and of a specific gravity of 1.03 was confirmed to be a meta-xylene solution of 29.9 mass % HFA·W. By NMR (¹³C, H and F) and FT-IR measurements, it was confirmed that only hexafluoroacetone monohydrate (1,1,1,3,3,3-hexafluoropropane-2-diol) existed in meta-xylene. Furthermore, it was confirmed by measuring moisture with a Karl Fischer moisture measurement device that the ratio of HFA to water was 1:1. Yield of hexafluoroacetone monohydrate by the present dehydration method was 70.4%.

## Claims

1. A process for producing hexafluoroacetone monohydrate, comprising introducing a hexafluoroacetone hydrate and an organic solvent into a distillation column after a previous mixing of them or separately, obtaining by azeotropic distillation (a) a composition containing the organic solvent and water as a low-boiling-point component from a column top, and (b) a composition containing hexafluoroacetone monohydrate and the organic solvent as a high-boiling-point component from a column bottom.

2. A process for producing hexafluoroacetone monohydrate according to claim 1, wherein at least one of the hexafluoroacetone hydrate and the organic solvent is continuously introduced into the distillation column.

3. A process for producing hexafluoroacetone monohydrate according to claim 1 or 2, wherein at least one of the compositions obtained from the column bottom or the column top is continuously taken out of the distillation column.

4. A process for producing hexafluoroacetone monohydrate according to any one of claims 1-3, wherein the hexafluoroacetone hydrate is hexafluoroacetone trihydrate (HFA·3W).

5. A process for producing hexafluoroacetone monohydrate according to claim 1, wherein an azeotrope of the hexafluoroacetone monohydrate and the organic solvent is obtained from the composition containing the hexafluoroacetone monohydrate and the organic solvent, which is obtained from the column bottom.

6. A process for producing hexafluoroacetone monohydrate according to any one of claims 1-5, wherein the organic solvent is an aromatic compound or ether compound.

## Patentansprüche

1. Verfahren zum Herstellen von Hexafluoracetonmonohydrat, umfassend Einleiten von Hexafluoracetonhydrat und organischem Lösungsmittel in eine Destillationskolonne nach vorhergehendem Mischen derselben oder getrennt und Erhalt (a) einer Zusammensetzung, die das organische Lösungsmittel und Wasser enthält, als Komponente mit niedrigem Siedepunkt von einem Kolonnenkopf und (b) einer Zusammensetzung, die Hexafluoracetonmonohydrat und das organische Lösungsmittel enthält, als Komponente mit hohem Siedepunkt von einem Kolonnenboden durch azeotrope Destillation.

2. Verfahren zum Herstellen von Hexafluoracetonmonohydrat nach Anspruch 1, wobei das Hexafluoracetonhydrat und/oder das organische Lösungsmittel stetig in die Destillationskolonne eingeleitet wird/werden.

3. Verfahren zum Herstellen von Hexafluoracetonmonohydrat nach Anspruch 1 oder 2, wobei zumindest eine der Zusammensetzungen, die von dem Kolonnenboden oder dem Kolonnenkopf erhalten werden, stetig aus der Destillationskolonne entnommen wird.

4. Verfahren zum Herstellen von Hexafluoracetonmonohydrat nach einem der Ansprüche 1 bis 3, wobei das Hexafluoracetonhydrat Hexafluoracetontrihydrat (HFA·3W) ist.

5. Verfahren zum Herstellen von Hexafluoracetonmonohydrat nach Anspruch 1, wobei ein Azeotrop des Hexafluoracetonmonohydrats und des organischen Lösungsmittels aus der Zusammensetzung erhalten wird, die das Hexafluoracetonmonohydrat und das organische Lösungsmittel enthält und welche von dem Kolonnenboden erhalten wird.

6. Verfahren zum Herstellen von Hexafluoracetonmonohydrat nach einem der Ansprüche 1 bis 5, wobei das organische Lösungsmittel eine aromatische Verbindung oder Etherverbindung ist.

## Revendications

1. Procédé de préparation du monohydrate d'hexafluoroacétone, comprenant l'introduction d'un hydrate d'hexafluoroacétone et d'un solvant organique dans une colonne de distillation après un mélange préalable de ceux-ci ou bien séparément, l'obtention par distillation azéotrope (a) d'une composition contenant le solvant organique et de l'eau comme composant à bas point d'ébullition depuis un haut de colonne, et (b) d'une composition contenant du monohydrate d'hexafluoroacétone et le solvant organique comme composant à point d'ébullition élevé depuis un bas de colonne.

2. Procédé de préparation du monohydrate d'hexafluoroacétone selon la revendication 1, dans lequel au moins l'un parmi l'hydrate d'hexafluoroacétone et le solvant organique est introduit en continu dans la colonne de distillation.

3. Procédé de préparation du monohydrate d'hexafluoroacétone selon la revendication 1 ou 2, dans lequel au moins l'une des compositions obtenues à partir du bas de colonne ou du haut de colonne est prélevée de la colonne de distillation en continu.

4. Procédé de préparation du monohydrate d'hexafluoroacétone selon l'une quelconque des revendications 1 à 3, dans lequel l'hydrate d'hexafluoroacétone est le trihydrate d'hexafluoroacétone (HFA.3W).

5. Procédé de préparation du monohydrate d'hexafluoroacétone selon la revendication 1, dans lequel un azéotrope du monohydrate d'hexafluoroacétone et du solvant organique est obtenu à partir de la composition contenant du monohydrate d'hexafluoroacétone et du solvant organique, qui est obtenue à partir du bas de colonne.

6. Procédé de préparation du monohydrate d'hexafluoroacétone selon l'une quelconque des revendications 1 à 5, dans lequel le solvant organique est un composé aromatique ou un composé éther.
